# EUROPEAN PATENT SPECIFICATION

(11) **EP 3 506 768 B1**
(45) Date of publication and mention of the grant of the patent: **07.01.2026**
(21) Application number: 17755546.3
(22) Date of filing: 29.08.2017
(51) Int. Cl.: A23K 20/163, A23K 50/10, A23K 50/60

(54) **MILK REPLACER COMPOSITION COMPRISING 2'-FUCOSYLLACTOSE FOR USE IN THE TREATMENT OR PREVENTION OF GASTRO-INTESTINAL DISEASES OF PRODUCTION ANIMALS**
MILCHERSATZZUSAMMENSETZUNG, UMFASSEND 2'-FUCOSYLLACTOSE ZUR VERWENDUNG BEI DER BEHANDLUNG ODER PRÄVENTION VON GASTROINTESTINALEN ERKRANKUNGEN VON PRODUKTIONSTIEREN
COMPOSITION DE SUBSTITUT DE LAIT COMPRENANT DU 2'-FUCOSYLLACTOSE POUR UNE UTILISATION DANS LE TRAITEMENT OU LA PRÉVENTION DE MALADIES GASTRO-INTESTINALES D'ANIMAUX DE PRODUCTION

(30) Priority: 31.08.2016 EP 16186533
(43) Date of publication of application: 10.07.2019
(73) Proprietor: OligoScience Biotechnology GmbH, 48341 Altenberge (DE)
(72) Inventor: PERACHA, Max, 59387 Ascheberg (DE); WEMHOFF, Sabrina, 79387 Ascheberg (DE)
(74) Representative: WSL Patentanwälte Partnerschaft mbB
(86) International application number: PCT/EP2017/071606
(87) International publication number: WO 2018/041803

(56) References cited:
- WO-A1-2010/120682
- WO-A1-2012/158517
- WO-A1-2015/071401
- US-A1- 2013 315 990
- S MARTÍN-SOSA ET AL: "Sialoglycoconjugate content of milk replacers for neonatal calves", SPANISH JOURNAL OF AGRICULTURAL RESEARCH, vol. 7, no. 2, 1 June 2009 (2009-06-01), ES, pages 322 - 329, XP055416450, ISSN: 1695-971X, DOI: 10.5424/sjar/2009072-423

## Description

The present disclosure relates to the use of human milk oligosaccharides for feeding production animals such as calves. The present invention relates to milk replacer compositions containing 2'-fucosyllactose (2-FL) for preventing and treating gastro-intestinal diseases, particularly gastro-intestinal infections, of young production animals, in particular calves.

The raising of healthy calves is the basis for successful dairy farming. Healthy calves obtained through optimal farming and care, an early development of the function of the gastroesophageal vestibule and an economically sensible raising are several of the objects of calf raising (Steinwunder et al. (2005) Milchviehfütterung, Tier- und Leistungsgerecht. Leopold Stocker Verlag, Graz; Drochner, W. (2008): Fütterung der Rinder. in: Jeroch et al. (eds.) Ernährung landwirtschaftlicher Nutztiere, 2nd ed. Eugen Ulmer KG, Stuttgart, pp. 385-467).

A successful raising is connected to the minimization of animal lost, which is regarded as the most crucial problem. In Germany, raising lost has been in the range of 8 to 14 % for several years (see, for example, Röhrmoser (1995): Aufzucht des Rindes. in: Matzke et al. (eds.) Wirtschaftliche Milchviehhaltung und Rindermast. 3rd ed., Frankfurt am Main, Verlag Union Agrar, pp. 221-261). The goal should be to attain a lost of around 5 %. Optimized calf feeding can contribute to this goal. Several prophylactic measures have been suggested in the context of feeding, for example to reduce instances of calf diarrhea (Schrag et al. (1984) Gesunde Kälber - Gesunde Rinder. Die wichtigsten Krankheiten in Aufzucht und Mast. Erkennung, Vorbeuge, Behandlung. 3rd ed. Henbersberg, Schober Verlags-GmbH, pp 249-276). The focus of these measures is a well-developed gut microflora and healthiness of the intestine. Various prebiotics have been suggested for inclusion into the milk replacer so as to improve and sustain gut healthiness (Dohms (2004) Aspekte der Darmgesundheit und Chancen für den Einsatz von Probiotika, 4th ed.).

Human milk oligosaccharides (HMOs) are a family of diverse unconjugated glycans that are abundant and unique to human milk; reviewed, e.g. in Bode (2012) Glycobiology 22 (9), pp. 1147-1162.

WO-A-2012/158517 discloses the use of human milk oligosaccharides, in particular 2'-fucosyllactose for the promotion of growth of bacteria found in infant faeces. This prior art also intends to use human milk oligosaccharides as prebiotics for nutrition of diverse mammalia, however, besides growth promotion of enterobacteria, no experimental data whatsoever are presented so that the use of human milk oligosaccharides for feeding animals remains speculative. WO 2010/120682 discloses a food composition for animal consumption, which includes an animal foodstuff and a milk-derived oligosaccharide or a glycoconjugate containing the oligosaccharide. The authors compared the effects of (a) whole human milk, (b) skim human milk, (c) total proteins isolated from human milk, and (d) oligosaccharides purified from human milk, in suppressing the growth of *Clostridium perfringens.*

The technical problem underlying the present invention is to provide improved raising of young production animals, in particular calves, especially through improvement of their feeding.

The solution to the above technical problem is provided by the embodiments of the present invention as described herein and as defined in the claims.

In particular, the present invention relates to novel milk replacer compositions containing 2'-fucosyllactose (2-FL).

According to the disclosure, a "human milk oligosaccharide" is a sugar molecule composed of at least three carbohydrate entities, which sugar molecule is found in the milk or pre-milk of female humans. Preferably, the HMO is an oligosaccharide composed of 3, 4, 5, 6, 7, 8, 9, 10 or more sugar entities, whereby oligosaccharides containing 4 or more entities may be linear or branched oligomers. HMOs are typically composed of the monosaccharides glucose, galactose, N- acetyglucosamine, N-acetylcalactosamine, fucose and N-acetylneuraminic acid. Most HMOs for use in the disclosure are based on the disaccharides lactose or N- acetyllactosamine, with those based on lactose are particularly preferred. Preferred human milk oligosaccharides in the context of the disclosure include, but are not limited to, 2'-fucoyllactose (2-FL), 3'-fucosyllactose (3-FL), 3'-sialyllactose (3-SL), 6'-sialyllactose (6-SL), lacto-N-tetraose (LNT), lacto-N-neotetraose (LNnT), lacto-N- hexaose (LNH), *iso*-lacto-N-octaose, *iso*-lacto-N-neooctaose, *para*-lacto-N-octaose, lacto-N-fucopentaose I (LNFP I), lacto-N-fucopentaose II (LNFP II), lacto-N- fucopentaose III (LNFP III), lacto-N-fucopentaose V (LNFP V), LS-tetrasaccharide a (LST a), LS-zetrasaccharide b (LST b), LS-zetrasaccharide c (LST c) and disialyllacto-N-tetraose (DSLNT).

Particularly preferred HMOs for use in the present disclosure are the human milk trisaccharides 3-FL and 2-FL. Recently, a biotechnological production of 2-FL has been provided (see WO-A-2010/070104) so that this basic HMO is available on an industrial scale (Jennewein Biotechnologie GmbH, Rheinbreitbach, Germany). The 2-FL in the milk replacer composition is therefore a synthetic one that has been prepared in bacterial culture, especially using genetically modified bacteria as taught in WO-A-2010/070104. 2-FL, in particular synthetic 2-FL as described before, 20 and higher HMOs based on 2-FL are generally preferred in the context of the disclosure and 2-FL is the most preferred HMO for the practice of the present disclosure.

The present disclosure also relates to milk replacer compositions comprising more than one particular HMO whereby a mixture of two or more of the above-mentioned HMOs is preferred. Particularly preferred is a mixture of HMOs whereby the mixture comprises both 2-FL and 3-FL together with third, fourth, fifth etc. further HMOs. Most preferred in this context of the disclosure is a combination of 2-FL and 3-FL.

The milk replacer composition of the disclosure can, of course, contain other sugars in addition to HMOs. In this context, lactose and N-acetyllactosamine are to be mentioned as preferred further oligosaccharides.

A "milk replacer" or "milk replacer composition" means a nutritional composition for feeding of production animals, preferably selected from cattle, goat, sheep, pig, deer and horse, especially infant and young production animals. The term "milk replacer" and "milk replacer composition" according to the present invention also includes the definition pursuant to Art. 3 (2) of the Regulation (EC) No. 767/2009 of the European Parliament and the Council, according to which a "milk replacer" means a "compound feed administered in dry form or after dilution in a given quantity of liquid for feeding young animals as a complement to, or substitute for, post-colostral milk or for feeding young animals such as calves, lambs or kids intended for slaughter".

The milk replacer composition of the disclosure thus typically contains one or more HMOs as described above together with a basic milk replacer composition. The term milk replacer composition according to the invention comprises the composition in dry form as well as its liquid form which is typically obtained by reconstitution of the dry composition with water. Basic nutritional compositions for providing the milk replacer composition of the invention are known in the art (for an overview for calf milk replacers, see, e.g., Bovine Alliance on Management and Nutrition (BAMN), 2008, A GUIDE TO CALF MILK REPLACERS, available online at https://www.aphis.usda.gov/animal_health/nahms/dairy/downloads/bamn/BAMN08_GuideMilk Repl.pdf; and Kamphues et al. (2004) Supplemente zu Vorlesungen und Übungen in der Tierernährung, Hannover, Verlag M. & H. Schaper Alfred).

Milk replacer compositions according to the invention thus generally comprise at least a protein and a fat source, optionally reconstituted in an aqueous liquid (in which ingredients may be present in the aqueous base in the form of suspension, emulsion and/or solution), typically in admixture with further ingredients such as carbohydrates, fiber, vitamins, mineral supplements, and further additives such as medicaments, emulsifiers, thickeners, trace elements, antioxidants and flavorings.

Milk replacer compositions are usually categorized according to the source of their protein and/or fat constituents. Typical protein sources for milk replacer compositions, especially for use in calf feeding, include skimmed milk powder, whey powder, and plant protein sources such as soy protein, soy flour, wheat gluten or wheat isolate. Fat components of milk replacers may be from animal fat or vegetable oils.

Protein and fat levels are both important features of milk replacer compositions for use in the invention. Preferred protein levels in calf milk replacers typically range from about 18 to about 22% (w/w), most preferred about 20 % (w/w), and fat levels are preferably in the range of from about 10 to about 28 % (w/w), with about 18 to about 23% (w/w) fat being more preferred. The levels of ingredients also vary with the type of animal to be fed. For example, for breed calves the fat level typically ranges from about 13 to about 20 % (w/w), while the fat content in the milk replacer composition should be elevated to around 23 % (w/w) for fattening calves, i.e. calves intended for slaughter. Of special consideration for production of milk replacer compositions is also the ratio of carbohydrate to protein. For example, pure dried whey has a lactose content of about 70 % (w/w), and the protein content is about 12 % (w/w). For practicing the invention, the content of carbohydrates (excluding the HMO content), in particular lactose, glucose and saccharose, does not exceed a value of about 50 % (w/w), so as to prevent a high passage rate leading to misfermentations in the colon (Kamphues (2004), *supra*).

A typical basic milk replacer composition for use in the disclosure contains about 22 % (w/w) or more crude protein, about 20 % (w/w) or more crude fat, not more than about 0.15 (w/w) crude fiber, calcium in an amount of from about 0.75 % (w/w) to about 1.75 % (w/w), at least about 0.7 % (w/w) phosphorous, at least about 20000 IU/Ib Vitamin A, at least about 5000 IU/Ib Vitamin D3, and about 100 IU/Ib Vitamin E.

The milk replacer composition according to the invention typically contains 2-FL in an amount of from about 0.5 to 10.0 % (w/w), preferably about 1 to 5 % (w/w), most preferred 4 % (w/w), based on the total weight of the composition.

The present disclosure is also directed to the use of HMOs (one or more of HMOs) for feeding of production animals, in particular young production animals such as calves, lambkins, kids and foals. The HMOs are particularly useful in calves fattening.

The present disclosure is also directed to a method for feeding production animals, in particular young production animals such as calves, lambkins, kids and foals, comprising the step of feeding the production animal with a milk replacer composition of the disclosure. The feeding method of the disclosure improves the feed intake by and performance parameters of the production animals, especially in the context of calves fattening. A special benefit of using HMOs as an ingredient in milk replacer compositions as taught herein is an improvement of overall conditions of the fed production animals, in particular calves, lambkins, kids and foals, so that the performance parameters of the animal fed with a milk replacer according to the disclosure improve. Milk replacer compositions according to the disclosure decrease animal lost, especially by preventing gastro-intestinal diseases such as bacterial or viral gut infections leading to diarrhea. Without wishing to be bound to any theory, it is believed that the HMOs improve the growth of favorable intestinal bacteria thereby reducing the relative number of pathogenic bacteria, while at the same time prevent or at least hinder binding of pathogenic agents such as pathogenic enterobacteria and viruses to intestinal receptors. The HMOs are also believed to improve the animal's gut microflora, modulate muccosal immunity, and strengthen the animal's resistance against atopic conditions.

In the context of the present disclosure, the HMOs are particularly useful in improving the healthiness of production animals such as calves, lambkins, kids and foals. In particular, the HMOs (which can be either given alone or it can be provided as a mixture of HMOs, as described above) are used for prevention and treatment, and at least reduce the incidents of, gastro-intestinal diseases, in particular bacterial and viral gastro-intestinal diseases such as diarrhea (or at least shorten the periods of such diseases) in said production animals. The HMOs also are useful in preventing and treating atopic diseases, i.e. conditions associated with a hypersensitivity for substances foreign to the body, in production animals, preferably those as defined above. Furthermore, the HMOs are useful for improving the mucosal immunity of production animals, in particular those mentioned before. According to the disclosure, the HMOs also improve the intestinal flora of the production animals such as calves, lambkins, kids and foals. The HMOs can be administered alone or in form of a composition, preferably in oral administration form, which can also take the form of a milk replacer composition as defined herein. The medical composition can also take the form of a tablet or capsule given separately from any feeding composition. The disclosure is therefore also directed to a method for preventing or treating a gastro- intestinal disease such as bacterial and viral infection in the gastro-intestinal system, for example diarrhea, in a production animal, preferably a young production animal as outlined above, comprising the step of administering a safe and effective amount of at least one HMO, or a composition containing two or more HMOs, to the production animal. The present disclosure is furthermore directed to the use of at least one HMO, or of a composition containing two or more HMOs, for the production of a medicament for the prevention or treatment of the above-mentioned conditions.

The present invention is further illustrated by the following non-limiting examples: EXAMPLES

### Example 1: Feeding of breed calves using milk replacer containing HMO

Materials and methods: 60 calves (Deutsche Holstein), are divided into two groups (control group and experimental group, 30 each) with equal numbers of male (15/30) and female (15/30) animals. The average age of the calves is 7 days. Each animal in both groups is held in single keeping. At the age of 14 days, the calves are held in the two groups with each group held in a different, but otherwise identical stable. The animals are fed with the milk replacer composition via an automated watering dispenser. The animals can take water freely from a water dispenser. In addition, 150 g of hay is given to each animal per day. From the 8^{th} week onwards, concentrated feed is given via an automated dispenser. Both groups, except for HMOs, are fed with the same milk replacer compositions. The experimental group is fed with basic milk replacer composition plus 4 % (w/w) 2-FL. The control group is fed with additional 4 % (w/w) whey powder instead of the HMO. One litre of the liquid milk replacer composition contains 125 g dry milk replacer concentrate.

The following table shows the feeding plan:

| **Age** | **Feed** | **Volume [I]** | **Stable** |
|---|---|---|---|
| First week | Colostrum | | Single |
| Second week | 2 x 3 I milk replacer or milk replacer + HMO | 42 | Single |
| 3^{rd} to 7^{th} week | 2 x 3 I milk replacer or milk replacer + HMO | 210 | Group |
| 8^{th} to 10^{th} week | 2 x 2 I milk replacer or milk replacer + HMO | 56 | Group |
| 10 to 12^{th} week | 1 x 3 I milk replacer or milk replacer + HMO | 28 | Group |
| | | Total: 336 | |

| | | | |
|---|---|---|---|
| Duration of experiment: 77 days | | | |

Data collection: Feeding via automated dispenser with single animal recognition, measurement of food intake by each individual; hay and feed concentrate data are collected by weighing; daily weighing of each animal; sublingual measurement of body temperature at automated feeding dispenser, with rectal measurement once weekly for comparison and control; control of health condition and use of medication; weekly analysis of faeces with respect to colonisation by intestinal bacteria.

Feed analysis. analyses of milk replacer, hay and feed concentrate.

### Example 2: Feeding of fattening calves using milk replacer containing HMO

Materials and methods: 60 calves of the breed Fleckvieh, devided into two groups (control/experimental) with 30 calves each. Equal amounts of male (15/30) and female (15/30) calves in both groups. The average age of the animals is 14 days (50 to 60 kg live weight). The animals of each group are held in a stable, both stables are identical. The animals are fed with the milk replacer composition via an automated watering dispenser. The animals can take water freely. In addition, 300 g of hay is given to each animal per day. Both groups, except for HMOs, are fed with the same milk replacer compositions. The experimental group is fed with basic milk replacer composition plus 4 % (w/w) 2-FL. The control group is fed with additional 4 % (w/w) whey powder instead of the HMO. On the onset of the experiment, one litre of the liquid feed composition contains 125 g dry milk replacer concentrate. The concentration of the liquid feed composition is increased to 250 g per litre at the end of the experiment.

The feeding plan is as follows:

| **Age** | **Feed** | **Stable** |
|---|---|---|
| From 3^{rd} week | 6 I per animal and day (milk replacer or milk replacer plus HMO) increased to 16 I per animal and day | Group |
| 22^{nd} week | Slaughter | |

Duration of experiment: the experiment starts at the age of ca. 14 days and ends with slaughter at a weight of ca. 230 to 240 kg (ca. 22^{nd} week of experiment), resulting in a duration of ca. 140 days.

Data collection: Feeding via automated dispenser with single animal recognition, measurement of food intake by each individual; hey and feed concentrate data are collected by weighing; daily weighing of each animal; sublingual measurement of body temperature at automated feeding dispenser, with rectal measurement once weekly for comparison and control; control of health condition and use of medication; weekly analysis of faeces with respect to colonisation by intestinal bacteria. After slaughtering, the body of each animal is evaluated.

Feed analysis. analyses of milk replacer and hay.

## Claims

1. Milk replacer composition containing 2'-fucosyllactose for use in the treatment or prevention of gastro-intestinal diseases of production animals by improving the growth of favorable intestinal bacteria thereby reducing the relative number of pathogenic bacteria and preventing or at least hindering binding of pathogenic enterobacteria to intestinal receptors, wherein the 2'-fucosyllactose is synthetic 2'-fucosyllactose prepared in bacterial culture, and wherein the composition has a content of carbohydrates, excluding human milk oligo-saccharide, of no more than 50 wt%.

2. The composition for use according to claim 1, wherein the production animal is a calf, lambkin, goat, sheep, pig, deer, horse, kid or foal.

3. The composition for use according to claim 1 or 2, wherein the method is for feeding a calf for fattening.

4. The composition for use according to any one of claims 1 to 3, wherein the composition contains 2'-fucosyllactose in an amount of from 0.5 to 10.0 wt.-% based on the total weight of the composition.

5. The composition for use according to e any one of claims 1 to 4, wherein the composition contains 2'-fucosyllactose in an amount of from 1.0 to 5.0 wt.-% based on the total weight of the composition.

6. The composition for use according to any one of claims 1 to 5, wherein the composition contains 2'-fucosyllactose in an amount of 4.0 wt.-% based on the total weight of the composition.

## Patentansprüche

1. Zusammensetzung eines Milchersatzes enthaltend 2'-Fucosyllactose zur Verwendung in der Behandlung oder Vorbeugung von gastrointestinalen Erkrankungen von Produktionstieren durch Verbessern des Wachstums von günstigen Darmbakterien, wodurch die relative Anzahl pathogener Bakterien reduziert wird und das verhindert oder zumindest hindern einer Bindung von pathogenen Enterobakterien an Darmrezeptoren, wobei die 2'-Fucosyllactose synthetische 2'-Fucosyllactose ist, die in einer Bakterienkultur hergestellt wurde, und wobei die Zusammensetzung einen Gehalt an Kohlenhydraten, ohne humane Milch-Oligosaccharide, von nicht mehr als 50 Ma.-% hat.

2. Die Zusammensetzung zur Verwendung nach Anspruch 1, wobei das Produktionstier ein Kalb, Lamm, Ziege, Schaf, Schwein, Hirsch, Pferd, Zicklein oder Fohlen ist.

3. Die Zusammensetzung zur Verwendung nach Anspruch 1 oder 2, wobei das Verfahren zum Füttern eines Kalbes zur Mast bestimmt ist.

4. Die Zusammensetzung zur Verwendung nach einem der Ansprüche 1 bis 3, wobei die Zusammensetzung 2'-Fucosyllactose in einer Menge von 0.5 bis 10.0 Ma.-% bezogen auf das Gesamtgewicht der Zusammensetzung enthält.

5. Die Zusammensetzung zur Verwendung nach einem der Ansprüche 1 bis 4, wobei die Zusammensetzung 2'-Fucosyllactose in einer Menge von 1.0 bis 5.0 Ma.-% bezogen auf das Gesamtgewicht der Zusammensetzung enthält.

6. Die Zusammensetzung zur Verwendung nach einem der Ansprüche 1 bis 5, wobei die Zusammensetzung 2'-Fucosyllactose in einer Menge von 4.0 Ma.-% bezogen auf das Gesamtgewicht der Zusammensetzung enthält.

## Revendications

1. Composition de substitut de lait contenant du 2'-fucosyllactose, à utiliser pour le traitement ou la prévention des maladies gastro-intestinales chez les animaux d'élevage, par amélioration de la croissance des bactéries intestinales bénéfiques, réduisant ainsi le nombre relatif de bactéries pathogènes et empêchant ou du moins entravant la liaison des entérobactéries pathogènes aux récepteurs intestinaux, où le 2'-fucosyllactose est du 2'-fucosyllactose synthétique préparé dans une culture bactérienne, et où la composition a une teneur en glucides, à l'exclusion des oligosaccharides du lait maternel, ne dépassant pas 50 % en poids.

2. Composition à utiliser selon la revendication 1, où l'animal d'élevage est un veau, un agneau, une chèvre, un mouton, un porc, un cerf, un cheval, un chevreau ou un poulain.

3. Composition à utiliser selon la revendication 1 ou 2, où la méthode consiste à nourrir un veau destiné à l'engraissement.

4. Composition à utiliser selon l'une quelconque des revendications 1 à 3, ladite composition contenant du 2'-fucosyllactose à raison de 0,5 à 10,0 % en poids basé sur le poids total de la composition.

5. Composition à utiliser selon l'une quelconque des revendications 1 à 4, ladite composition contenant du 2'-fucosyllactose à raison de 1,0 à 5,0 % en poids basé sur le poids total de la composition.

6. Composition à utiliser selon l'une quelconque des revendications 1 à 5, ladite composition contenant du 2'-fucosyllactose à raison de 4,0 % en poids basé sur le poids total de la composition.
